# EUROPEAN PATENT SPECIFICATION

(11) **EP 3 203 237 B1**
(45) Date of publication and mention of the grant of the patent: **05.07.2023**
(21) Application number: 16181112.0
(22) Date of filing: 25.07.2016
(51) Int. Cl.: G01N 33/569

(54) **URINE-DERIVED EPITHELIAL CELL LINES FOR DIAGNOSIS AND THERAPY OF AN ANTI-BK-VIRUS OR ANTI-GRAFT IMMUNE RESPONSE**
AUS URIN ABGELEITETE EPITHELZELLLINIEN ZUR DIAGNOSE UND THERAPIE EINER IMMUNANTWORT AUF BK-VIRUS ODER EINER TRANSPLANTAT-ABSTOSSUNGSREAKTION
LIGNÉES CELLULAIRES ÉPITHÉLIALES DÉRIVÉES DE L'URINE POUR DIAGNOSTIC ET THÉRAPIE DE LA RÉPONSE IMMUNITAIRE CONTRE LE VIRUS BK OU LE TRANSPLANT

(30) Priority: 03.02.2016 EP 16154152
(43) Date of publication of application: 09.08.2017
(73) Proprietor: Babel, Nina, 13469 Berlin (DE); Reinke, Petra, 10117 Berlin (DE); Thieme, Constantin, 13351 Berlin (DE); Weist, Benjamin, 10965 Berlin (DE)
(72) Inventor: Babel, Nina, 13469 Berlin (DE); Reinke, Petra, 10117 Berlin (DE); Thieme, Constantin, 13351 Berlin (DE); Weist, Benjamin, 10965 Berlin (DE)
(74) Representative: Schulz Junghans Patentanwälte PartGmbB

(56) References cited:
- WO-A1-2011/134989
- WO-A1-2016/073595
- B. J. D. WEIST ET AL: "The role of CD4+ T cells in BKV-specific T cell immunity", MEDICAL MICROBIOLOGY AND IMMUNOLOGY, vol. 203, no. 6, 23 July 2014 (2014-07-23) , pages 395-408, XP055338999, DE ISSN: 0300-8584, DOI: 10.1007/s00430-014-0348-z
- P. COMOLI: "Dendritic Cells Pulsed with Polyomavirus BK Antigen Induce Ex Vivo Polyoma BK Virus-Specific Cytotoxic T-Cell Lines in Seropositive Healthy Individuals and Renal Transplant Recipients", JOURNAL OF THE AMERICAN SOCIETY OF NEPHROLOGY., vol. 14, no. 12, 1 December 2003 (2003-12-01), pages 3197-3204, XP055255878, US ISSN: 1046-6673, DOI: 10.1097/01.ASN.0000096374.08473.E3
- EMILY BLYTH ET AL: "BK Virus-Specific T Cells for Use in Cellular Therapy Show Specificity to Multiple Antigens and Polyfunctional Cytokine Responses", TRANSPLANTATION, vol. 92, no. 10, 1 November 2011 (2011-11-01), pages 1077-1084, XP055034458, ISSN: 0041-1337, DOI: 10.1097/TP.0b013e31823328c0
- GIOVANNI B FOGAZZI ET AL: "Images in Nephrology: "Decoy cells" in the urine due to polyomavirus BK infection: easily seen by phase-contrast microscopy", NEPHROL DIAL TRANSPLANT, vol. 16, no. 7, 1 July 2001 (2001-07-01), pages 1496-1498, XP055342638,
- HOWARD E COREY: "Practical pediatric nephrology Urine cytology: an underused method to diagnose acute renal allograft rejection", PEDIATRIC NEPHROLOGY, vol. 11, no. 2, 1 April 1997 (1997-04-01), pages 226-230, XP055343707,

## Description

### Field of the invention

The present invention relates to a method for monitoring of an immune response in a patient after kidney transplantation.

### Background of the invention

Two of the most serious immune-related complications after kidney transplantation are graft-rejection due to alloreactive immune cells and BK-virus associated nephropathy (BKVAN).

Graft-rejection is the result of a response of the cellular immune system, in particular T-cells, against the transplanted donor tissue and is a common complication for all types of organ transplants. Recipients of organ transplants are therefore usually treated with immunosuppressive drugs to limit the immune response of the host immune system to the donor tissue.

BK-virus associated nephropathy is the result of a BK-virus infection of the transplanted kidney. In immunocompetent individuals replication of the BK-virus is efficiently controlled by the immune system and does not cause any symptoms. However, in immunosuppressed kidney transplant patients BK-virus infection can cause BKVAN, which can result in the loss of the kidney transplant. There is no efficient antiviral therapy for BKV infection up to now. It is therefore imperative to monitor the immune response of the host against the tissue graft and BKV as well as any signs of a BK-virus infection and to adjust the immunosuppressive therapy accordingly. An increase in alloreactive T-cells makes it necessary to increase the dosage of the immunosuppressive therapy whereas the presence of BK-virus infection is indicative of a dosage reduction.

Monitoring of T-cells reactive against tissue grafts is difficult because the variety of donor specific proteins/antigens that may be the target of T-cells is very large and specific for each donor. Methods known in the art to overcome this problem include the use of other donor tissue such as the spleen only for the purpose of monitoring alloreactive T-cells. However, this approach only provides a limited supply of donor specific cells and requires cost and labour intensive storage of the cell material. Additionally, in many cases this additional donor tissue is simply not available. Furthermore, the presence of T-cells reactive to a specific graft tissue such as the kidney would not be detectable with the use of other donor tissue as various tissues differ in their major histocompatibility complex molecule expression, for instance.

A known method to monitor BK-virus specific T-cells is the use of so-called overlapping peptide pools (OVP) for specific BK-virus proteins. These peptide pools theoretically contain every possible epitope of the respective proteins. Such BK-virus specific OVP libraries can be presented by antigen presenting cells and reactivity of immune cells of the graft-recipient against BKV can be determined. A limitation of this currently applied method is that the antigen presenting cells (APCs) are not donor cells. This is important because each individual has a specific set of major histocompatibility complex (MHC) proteins that present the antigenic peptides to immune cells. Different sets of MHC proteins present different epitopes to the immune cells. In consequence, recipient APCs will present different peptides (as compared to donor APC, unless recipient and donor have a perfectly matched genetic background) and subsequently may miss T-cells reactive against BK-virus antigens presented by the set of donor-MHC molecules.

The problem underlying the present invention is to provide the means for *ex vivo* monitoring and isolation of BK-virus reactive and alloreactive T-cells in a patient after kidney transplantation. This problem is solved by the subject-matter of the independent claims.

### Description of the invention

The invention is defined by the appended claims.

Kidney grafts, in particular the renal tubules, lose a significant amount of cells after transplantation, which are shed from the body with urine. These epithelial cells are known as renal proximal tubular epithelial cells (RPTEC) and can be grown in cell culture. Furthermore, RPTECs express functional MHC-class I and II molecules on their surface and are therefore suitable as antigen presenting cells for CD8⁺ and CD4⁺ T-cells. They comprise the full variety of donor proteins and have donor-identical MHC-molecules for representation of potential antigens to the recipient's immune cells and therefore represent a renewable and extendable source of donor specific antigen presenting cells.

Using the method of the present invention, a sample of whole blood obtained from a graft recipient can be screened for immune cells reactive against BK-virus infected donor tissue. RPTECs are infected *in vitro* with BK-virus and are used as stimulator for screening and obtaining of BKV-specific T-cells in whole blood of the patient. Described but not part of the invention is, that RPTEC can be loaded by BKV-specific OVP for screening of BKV-reactive T-cells.

The invention can further be used to identify and obtain T-cells directly or indirectly reactive against the graft tissue by using non-infected RPTECs from the same patient.

The finding was made that epithelial cells isolated from urine of patients after kidney transplantation can be used to monitor immune responses of the patient against BK-virus infection as well as against the donor tissue itself.

The first part of the first aspect of the invention relates to a method for detecting an immune response against BK virus in a patient after kidney transplantation. The method comprises the following steps:
a) A cell isolation step comprising:
   i. isolating epithelial cells from the urine of the patient, and
   ii. providing a sample of whole blood obtained from the patient.
b) A cell cultivation step comprising:
   i. cultivating the epithelial cells, thereby yielding cultivated epithelial cells, and
   ii. infecting the cultivated epithelial cells with BK-virus, yielding BK-virus infected epithelial cells.
c) A direct reactive T-cell determination step.

The presence of T-cells, particularly comprised within the sample obtained from the patient, that are activated by the BKV-infected epithelial cells is indicative of an immune response.

The presence of BK-virus reactive T-cells is indicative of a specific immune response. In the context of the present specification, only a reactive T-cell number higher than the number of non-specifically activated T-cells ("false positives") is regarded as "present". A non-limiting example of a method to determine the false positives ("background noise") is to determine the number of activated T-cells without contact to the specific target cells (epithelial cells, BKV-infected epithelial cells or loaded APCs).

In the context of this specification *contacting* the cultivated epithelial cells with BK-virus (BKV) refers to infecting the epithelial cells with BKV. Therefore, contacting the cells with BKV is performed under conditions suitable for a viral infection. In the context of the present specification the term *BKV-reactive T-cells* refers to T-cells that are able to specifically bind to cells of the tissue graft that are infected with BKV or at least present BKV antigens on its surface. Binding of those T-cells with their T-cell receptor results in activation of the BKV-reactive T-cells.

The presence of T-cells, particularly comprised within the sample obtained from the patient, that are activated by the BKV-infected epithelial cells is indicative of an immune response against BK virus.

The T-cell determination step comprises a direct reactive T-cell determination comprising:
i. contacting at least a part of the sample of whole blood of the patient with the BKV-infected epithelial cells, and
ii. determining T-cells comprised within the sample of whole blood that are activated by the BKV-infected epithelial cells, yielding a BKV-reactive T-cell number;

Non-limiting examples of methods suitable for determining and isolating T-cells are methods such as flow cytometry, fluorescence activated cell sorting (FACS) or magnetic bead associated cell sorting (MACS). Those methods can be used to measure markers expressed on or within the T-cells and to separate the targeted T-cells.

In the context of the present specification, the term *overlapping peptide pool (OVP)* refers to a collection of antigens or single antigens. An OVP specific for BK-virus refers to a collection of antigens characteristic for BKV. In certain embodiments, a BKV specific peptide pool consists of 15-mer peptides overlapped by 11 amino acids representing antigens specific for BKV. The OVP can be presented by antigen-presenting cells in an MHC I or MHC II context to activate the respective immune-cells. Reference is made to US patent application US2004106159 for complete description of synthetic peptide libraries comprising an overlapping peptide pool.

In the context of the present specification the term *antigen-presenting cells (APC)* is used in its meaning known in the art of immunology; it refers to a cell being able to display antigens in complex with the major histocompatibility complex (MHC) on the cell surface. Such a representation of an antigen makes them accessible for T-cells. The type of T-cell being able to recognize the antigen depends on the type of MHC molecule presenting the antigen. MHC class I molecules are for example able to interact with CD8⁺ T-cells whereas MHC class II molecules are able to interact with CD4⁺ cells. Non-limiting examples of APCs are dendritic cells, macrophages. The epithelial cells of the present invention are one example of a suitable APC in the context of the present invention.

In the context of the present specification a loaded antigen-presenting cell refers to an APC that has been exposed to antigenic molecules in such a way that these antigens are processed by the APC and presented in a MHC context.

In certain embodiments, in step a) PBMCs isolated from blood of the same patient are provided instead of said sample of whole blood. These PBMCs are used instead of the sample of whole blood in all subsequent steps.

In the second part of the first aspect the method additionally comprises:
a) in the cell isolation step:
   i. dividing the sample of whole blood in a first and a second portion of whole blood,
b) in the cell cultivation step:
   i.a dividing the cultivated epithelial cells into a first and a second portion of cultivated epithelial cells,
c) in the reactive T-cell determination step:
   - in case of a direct reactive T-cell determination:
      iii. contacting the second portion of whole blood with the second portion of cultivated epithelial cells and determining T-cells comprised within the second portion of whole blood that are activated by the second portion of cultivated epithelial cells, yielding an allograft reactive T-cell number,

The presence of reactive T-cells is indicative of an immune response, wherein graft reactive T-cells are indicative of an anti-graft immune response.

In the context of the present specification the term *allograft-reactive T-cells* refers to T-cells that are able to specifically recognize cells of the tissue graft and are activated by binding those allograft cells.

The method according to claim 1 comprises the following steps:
a) A cell isolation step comprising:
   i. isolating epithelial cells from the urine of the patient,
   ii. providing a sample of whole blood obtained from the same patient, and
   iii. dividing the sample of whole blood in a first and a second portion of whole blood.
b) A cell cultivation step comprising:
   i. cultivating the epithelial cells, thereby yielding cultivated epithelial cells, and
      i.a dividing the cultivated epithelial cells into a first and a second portion of cultivated epithelial cells
   ii. infecting the first portion of the cultivated epithelial cells with BK-virus, yielding BK-virus infected epithelial cells.
c) A direct reactive T-cell determination step comprising:
   i. contacting the first portion of whole blood of the patient with the BK-virus infected epithelial cells,
   ii. determining T-cells comprised within the first portion of whole blood that are activated by the BK-virus infected epithelial cells, thereby yielding a BK-virus-reactive T-cell number, and
   iii. contacting the second portion of whole blood with the second portion of cultivated epithelial cells and determining T-cells comprised within the second portion of whole blood that are activated by the second portion of cultivated epithelial cells, yielding a graft reactive T-cell number.

The presence of BK-reactive T-cells are indicative of an anti-BK-virus immune response and graft reactive T-cells are indicative of an anti-graft immune response.

In certain embodiments, the BKV-reactive T-cell number is higher than 0.001% of the number of all analysed T-cells (after background subtraction) to be considered present.

In certain embodiments, the allograft reactive T-cell number is higher than 0.001% of the number of all analysed T-cells (after background subtraction) to be considered present.

In certain embodiments, the epithelial cells isolated from urine in the cell isolation step:
- only comprises epithelial cells originating from the transplanted kidney of the patient (donor tissue) , and/or
- are not infected with BK-virus.

Isolation of epithelial cells originating from the renal tissue can be achieved by standard methods such as flow cytometry, magnetic bead associated cell sorting (MACS) and/or selection of cell clones. Therefore the cells isolated from urine would be separated according to the presence or absence of certain biomarkers specific for the graft-tissue or the graft-recipients tissue; specific biomarkers present on the surface of the cell are preferable. In certain embodiments single cell clones are selected from the urine derived cells and are cultivated. Those cell clones are used in certain embodiments to establish immortalised cell lines.

In certain embodiments, the T-cells are CD4 positive or CD8 positive T-cells.

According to a second aspect of the invention a diagnostic method for monitoring immunosuppressive therapy in a patient after kidney transplantation is provided. The diagnostic method comprises determination of allograft-reactive T-cells and BKV-reactive T-cells according to claim 1.

The presence of allograft-reactive T-cells is indicative of a necessary increase in immunosuppressive therapy. The presence of BKV-reactive T-cells in combination with the detection of a BK-virus infection in the patient is indicative of a necessary reduction in immunosuppressive therapy in the patient.

In other words, the absence of allograft-reactive T-cells in a patient is advantageous to prevent graft rejection. The presence of allograft-reactive T-cells is indicative for an increased risk of cellular rejection and an increase in immunosuppressive therapy to prevent graft-rejection is warranted. In contrast in a patient with a BK-virus infection the presence of BKV-reactive T-cells is advantageous to combat the BK-virus infection. The absence of BKV-reactive T-cells in a patient with a BK-virus infection (increased BKV load) would therefore be indicative of a necessary decrease in immunosuppressive therapy to allow an immune response against BK-virus.

In certain embodiments, allograft reactive T-cells are considered to be present if the allograft-reactive T-cell number is higher than 0.001% of the number of all analysed T-cells.

In certain embodiments, BKV-reactive T-cells are considered to be present if the BKV-reactive T-cell number is higher than 0.001% of the number of all analysed T-cells.

In certain embodiments, the allograft-reactive T-cell number and the BKV-reactive T-cell number are determined at a first and a second time point, wherein
- an increase in the allograft-reactive T-cell number between the first time point and the second time point is indicative of a necessary increase in immunosuppressive therapy and
- lack or decrease in BKV-reactive T-cell number between the first and the second timepoint in a patient with a BK-virus infection is indicative of a necessary reduction in immunosuppressive therapy.

In certain embodiments, the time between the first and the second time point is at least three days.

According to a third aspect of the invention an *in vitro* method for obtaining a T-cell from a patient after kidney transplantation is provided, wherein the T-cell is reactive against antigens derived from BK-virus or kidney graft tissue. The method comprises the following steps:
a) a cell isolation step comprising:
   i. isolating epithelial cells from the urine of the patient,
   ii. providing a sample of whole blood obtained from the patient,
b) a cell cultivation step comprising:
   i. cultivating the epithelial cells, yielding cultivated epithelial cells,
   ii. in case of the antigen being derived from BK-virus, contacting the cultivated epithelial cells with BK-Virus, yielding BKV-infected epithelial cells, c) a reactive T-cell stimulation step.

The reactive T-cell stimulation step comprises a direct reactive T-cell stimulation comprising the steps of:
i. contacting the sample of whole blood of said patient with:
   - the BKV-infected epithelial cells, in case of the antigen being derived from BK-virus, or
   - the cultivated epithelial cells, in case of the antigen being derived from kidney graft tissue,
   wherein the T-cell comprised within the sample of whole blood is stimulated with the respective antigen, yielding a stimulated T-cell,
ii. expanding the stimulated T-cell.

In certain embodiments, the obtained T-cell comprises the surface markers selected from:
i. CD4+CD25+Foxp3+CD137+CD154-,
ii. CD8+CD25+Foxp3+,
iii. CD4+RORgt+,
iv. CD4+IL22+IL17-,
v. CD4+CD137+CD154+,
vi. CD4+CD137+GranzymeB+, and
vii. CD8+CD137+GranzymeB+.

In certain embodiments, the obtained T-cell is used for the generation of T-cell lines.

Particularly, any sample comprising T cells obtained from the patient may be used instead of the sample of whole blood in the method according to any one of the above aspects or embodiments of the invention.

The invention is further illustrated by the following examples and figures, from which further embodiments and advantages can be drawn. These examples are meant to illustrate the invention but not to limit its scope.

### Short description of the figures

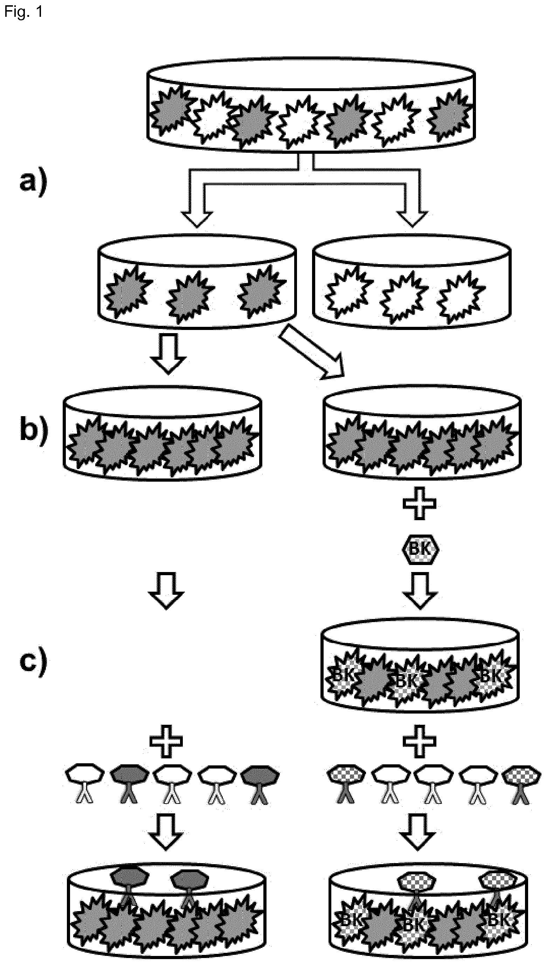

- Fig. 1: shows a scheme of the invention. a) Epithelial cells are isolated from urine of the patient and are separated into epithelial cells derived from graft (grey cells) and all other cells (white cells). b) cells are divided into separate cultures and are cultivated. One culture is infected with BK-virus yielding BK-virus infected epithelial cells (checkered cells). c) PBMCs (Y-cells) of the patient are added to the cultures of epithelial cells (infected as well as non-infected cultures) and T-cells reactive against graft cells (left side; grey Y-cells) and T-cells reactive against BK-virus infected cells (right side; checkered Y-cells) are determined.

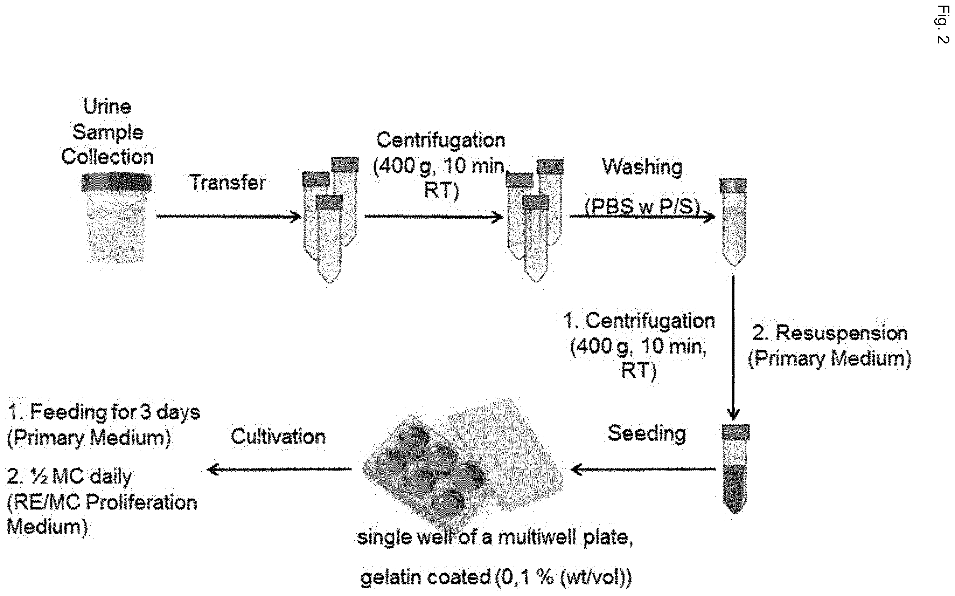

- Fig. 2: shows the isolation procedure of epithelial cells from the urine of a patient. Urine samples are centrifuged and washed with phosphate buffered saline (PBS) containing antibiotics (penicillin/streptomycin). After resuspension in primary growth medium the epithelial cells are seeded in multiwell plates. After three days of cultivation the primary medium is changed to a proliferation medium specific for renal epithelial cells (RE/MC).

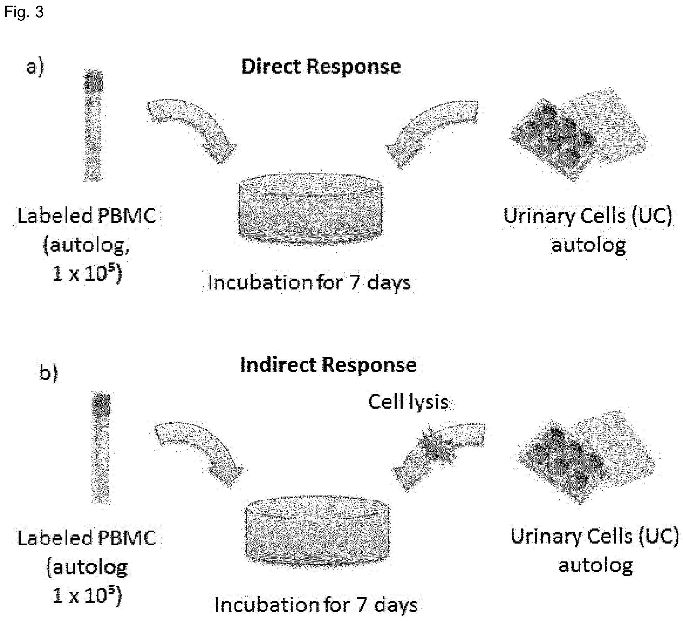

- Fig. 3: shows the general procedure of using the epithelial cells isolated from urine (urinary cell; UC) to a) directly or b) indirectly stimulate T-cells comprised in PBMCs of the patient, a) Labelled PBMCs and epithelial cells of the same patient were incubated together for seven days. The direct response of T-cells to the epithelial cells was analysed by FACS. b) Labelled PBMCs were incubated with antigen presenting cells of the patient that have been loaded with lysed epithelial cells. After seven days of incubation activation and proliferation of T-cells comprised within the PBMCs was measured by FACS. Donor-reactive cells can also be determined after short-term stimulation, using activation markers (CD154 and CD137) and effector cytokines (IFNγ, TNFα).

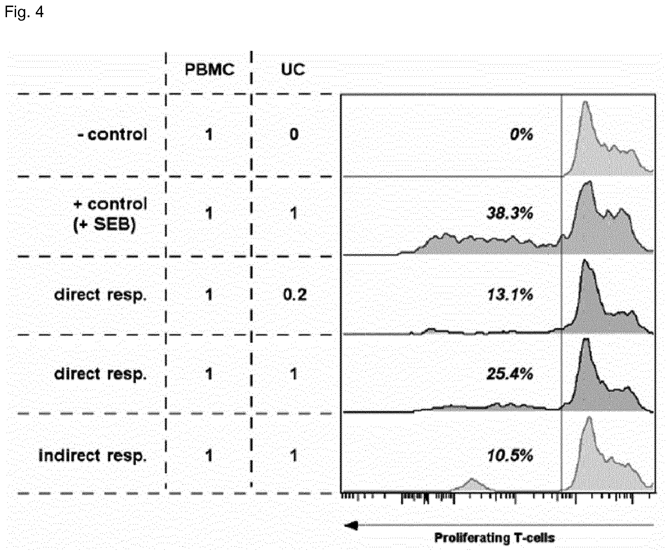

- Fig. 4: shows proliferation of graft-reactive T-cells comprised within PBMCs after direct or indirect exposure to epithelial cells (UC). Numbers provided in the columns "PBMC" and "UC" represent the proportions of PBMCs to UCs. Percentages of proliferating T-cells are indicated on the right-hand column. As positive control the PBMCs have been stimulated with staphylococcal enterotoxin B (SEB).

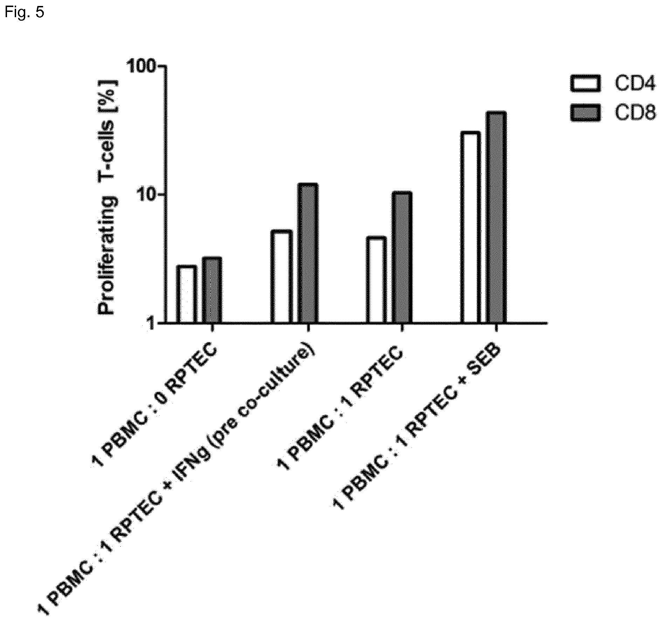

- Fig. 5: shows proliferation of graft-reactive CD4⁺ and CD8⁺ cells. PBMCs were cultivated with epithelial cells (RPTEC) in equal proportions and proliferating CD4⁺ and CD8⁺ T-cells were determined by FACS. As negative control PBMCs were incubated without epithelial cells (1 PBMC : 0 RPTEC) and as a positive control PBMCs were cultivated with RPTECs in the presence of staphylococcal enterotoxin B (1 PBMC : 1 RPTEC + SEB). Proliferation of CD4⁺ and CD8⁺ cells after co-cultivation of PBMCs with epithelial cells was measured with and without preincubation of RPTEC with interferon-gamma (IFNγ) to increase MHC antigen upregulation on RPTEC.

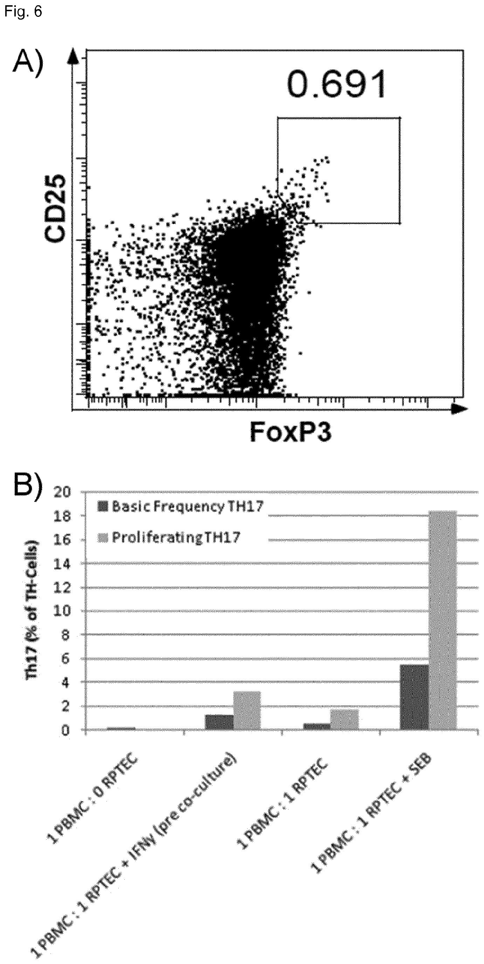

- Fig. 6: shows A) frequency of graft-reactive regulatory T-cells after 16 h of cocultivation of epithelial cells (RPTEC) and PBMCs and B) proliferation of Th17 cells after co-cultivation of epithelial cells (RPTEC) and PBMCs. As negative control PBMCs were incubated without epithelial cells (1 PBMC : 0 RPTEC) and as a positive control PBMCs were cultivated with RPTECs in the presence of staphylococcal enterotoxin B (1 PBMC : 1 RPTEC + SEB). Proliferation of Th17 cells after co-cultivation of PBMCs with epithelial cells was measured with and without preincubation of RPTEC with interferon-gamma (IFNg).

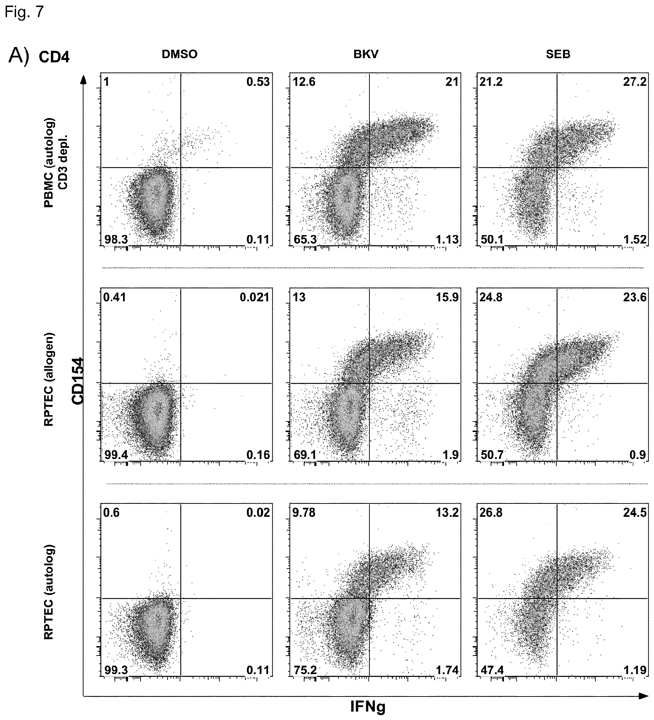

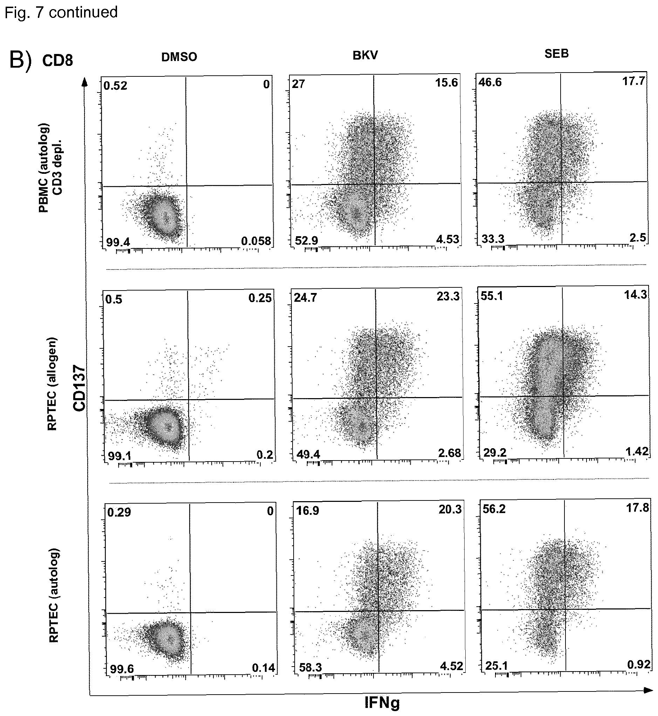

- Fig. 7: shows FACS analysis of A) reactive CD4⁺ T-cells and B) reactive CD8⁺ T-cells in response to allogenic or autologous epithelial cells (RPTECs) alone (left column) or presenting BK-virus specific peptides (middle column) after 4-7 days of co-culture. As negative control cells were incubated with DMSO only and as positive control staphylococcal enterotoxin B (SEB) was added. As markers of T-cell activation interferon-gamma production and CD154 (CD4⁺) or CD137 (CD8⁺) expression was measured. Presentation of BK-virus specific peptides activated CD4⁺ and CD8⁺ T-cells in all used types of antigen presenting cells (PBMC CD3 depleted; RPTEC allogenic, RPTEC autologous).

### Examples

### Example 1: Detection of a BK-virus immune response

The inventors made the finding that epithelial cells isolated from urine of patients after kidney transplantation can be used to monitor immune responses of the patient against BK-virus infection.

### Isolation of epithelial cells from the urine of a patient

Urine samples of patients having received a kidney transplantation were collected and centrifuged at 400g, for 10 minutes at room temperature to pellet all cells comprised in the urine sample (figure 2). Next, the resulting pellet was washed with phosphate buffered saline supplemented with antibiotics (e.g. peniciliin/streptomycin). After a second centrifugation step (400g, 10 minutes, room temperature) the cell pellet was resuspended in primary growth medium containing DMEM/Ham s F12 1:1 (Hyclone), 10 % of fetal bovine serum (FBS; PAA), SingleQuot Kit CC-4127 REGM (Lonza), amphotericin B and penicillin/streptomycin and seeded in gelatin coated multiwell plates. After three days of cultivation the medium was changed to a specific proliferation medium for renal epithelial cells such as EBM (Renal Epithelial Basal Medium, Lonza) medium containing SingleQuot Kit CC-4127 REGM (Lonza). Cultivated renal epithelial cells were then infected with BK-virus.

### Immune response against BK-virus infected epithelial cells

Peripheral blood mononuclear cells (PBMCs) were isolated from the patient and co-cultured with the BK-virus infected epithelial cells or BKV-OVP loaded epithelial cells in a short term stimulation (16h). The response of CD4⁺ and CD8⁺ T-cells against CD3-depleted autologous PBMCs and allogenic or autologous epithelial cells (RPTECs) presenting BK-virus specific peptides was measured by FACS analysis. Epithelial cells only treated with vehicle (DMSO) and not infected with BK-virus were used as negative control. Staphylococcal enterotoxin B (SEB) was used as a positive control to assess polyclonal reactivity of T-cells. As markers for the activation of CD4⁺ T-cells the presence of CD154 and the production of interferon-gamma (IFNg) were measured by FACS.

As shown in figure 7A the negative control (DMSO) only showed 0.53%, 0.021% and 0.02% for autologous PBMCs, allogenic RPTEC and autologous RPTEC, respectively. In contrast the positive control (SEB) resulted in the activation of 27.2%, 23.6% and 24.5% for the aforementioned cell types. Presentation of BK-virus specific peptides (BKV) by autologous PBMCs, allogenic RPTEC and autologous RPTEC activated 21%, 15.9% and 13.2% of the CD4⁺ T-cells.

Measurement of activated CD8⁺ cells revealed 0%, 0.25% and 0% for autologous PBMCs, allogenic RPTEC and autologous RPTEC in the negative control (DMSO). In contrast treatment with SEB (positive control) resulted in the activation of 17.7%, 14.3% and 17.8% of the CD8⁺ cells. An even higher level of activation was observed by stimulation with BK-virus peptide presenting autologous PBMCs, allogenic RPTEC and autologous RPTEC with 15.6%, 23.3% and 20.3% activation of CD8⁺ cells (figure 7B).

### Example 2: Detection of graft-reactive T-cells

The inventors made the finding that epithelial cells isolated from urine of patients after kidney transplantation can also be used to monitor immune responses of the patient against the donor graft tissue itself. This method can be used by itself to detect an anti-graft immune response and combined with the above mentioned method for the detection of BK-virus reactive T-cells to achieve information for the adjustment of an immunosuppressive therapy as claimed in the second aspect of the invention, or to provide differential diagnosis acute rejection vs. BKV associated nephropathy.

The isolation procedure of the epithelial cells from the urine of a patient having received a kidney transplant is identical with the method described above with the exception that the epithelial cells are not infected with BK-virus.

The activation by direct response to epithelial cells or APCs presenting peptides of the epithelial cells is performed as described below:
Peripheral blood mononuclear cells (PBMCs) were isolated from the patient and labelled with CFDA for the proliferation assay FACS analysis. CFDA-labelled PBMCs (1×10⁵ cells) were co-cultured with epithelial cells for seven days to test the direct graft-reactive response of T-cells comprised within the PBMCs against donor tissue (figure 3). Besides the direct presentation of MHC molecules, the MHC molecules can be indirectly presented on the recipient APC. For the indirect presentation, epithelial cells will be lysed and loaded on the recipient APC.

In figure 4 T-cells reactive against the graft tissue are shown. Specifically, the negative control (absence of epithelial cells (UC)) showed 0% activation of T-cells, whereas treatment with SEB (positive control) showed 38.3% activation of T-cells. Co-culture of autologous PBMCs with epithelial cells (UC) resulted in activation of 25.4% of T-cells, if PBMCs and UCs were present in equal proportions and only 13.1% of T-cells were activated, if PBMCs and UCs were present 5:1. Indirect presentation of graft-tissue peptides by APCs resulted in activation of 10.5% of the T-cells (figure 4).

In figure 5 proliferating CD4⁺ and CD8⁺ T-cells were measured in response to co-cultivation of PBMCs and epithelial cells (RPTEC) in equal proportions. As negative control PBMCs were incubated without epithelial cells (1 PBMC : 0 RPTEC) and as a positive control PBMCs were cultivated with RPTECs in the presence of staphylococcal enterotoxin B (1 PBMC : 1 RPTEC + SEB). Proliferation of CD4⁺ and CD8⁺ cells after co-cultivation of PBMCs with epithelial cells was measured with and without preincubation of RPTEC with interferon-gamma (IFNg). Treatment with SEB (positive control) resulted in the proliferation of 50% to 60% of CD4⁺ and CD8⁺ T-cells. The co-culture of PBMCs and RPTEC with or without IFNg pretreatment resulted in the proliferation of ~5-6% of CD4⁺ cells and ~10 to 15% of CD8⁺ cells.

In figure 6 A) the frequency of graft-reactive regulatory T-cells were measured in response to 16 h of co-cultivation of epithelial cells (RPTEC) and PBMCs. The expression of CD25 and FoxP3 was determined as markers of activated regulatory T-cells. Figure 6 B) shows the proliferation of Th17 cells after co-cultivation of epithelial cells (RPTEC) and PBMCs. As negative control PBMCs were incubated without epithelial cells (1 PBMC : 0 RPTEC) and as a positive control PBMCs were cultivated with RPTECs in the presence of staphylococcal enterotoxin B (1 PBMC : 1 RPTEC + SEB). Proliferation of Th17 cells after co-cultivation of PBMCs with epithelial cells was measured with and without preincubation of RPTEC with interferon-gamma (IFNg).

### Example 3: Diagnostic method for monitoring immunosuppressive therapy in a patient after kidney transplantation.

The transplantation of kidney-grafts is in many cases the only available treatment option for patients experiencing kidney failure. In order to avoid graft-rejection the patient has to be treated with immunosuppressive drugs to limit the immune response against the donor tissue. The immunosuppressive therapy has to be monitored closely and adjusted regularly to avoid other complications. A very common and serious side effect of immunosuppressive therapy after kidney transplantation is a BK-virus infection of the transplanted kidney. While BK-virus infection is efficiently controlled by the immune system of immunocompetent individuals and usually is without any noticeable symptoms, in immunosuppressed kidney transplant patient BK-virus infection can result in BK-virus associated nephropathy (BKVAN). BKVAN can also lead to the loss of the kidney transplant and therefore immunosuppressive therapy has to be finely adjusted to find a balance between suppression of graft-rejection and control of BK-virus infection. Therefore, the immune response of the patient against the donor tissue and the occurrence of BK-virus infection within the donor tissue have to be monitored closely. The above mentioned methods of this invention provides the means to detect an immune response directed against BK-virus infection of the donor tissue as well as an immune response against the donor tissue itself. An increase in alloreactive T-cells would make it necessary to increase the dosage of the immunosuppressive therapy whereas the presence of BK-virus infection and the lack of BKV-specific T-cells would be indicative of a dosage reduction.

### Example 4: Generation of BKV-specific effector T-cells and graft-reactive regulatory T-cells for the treatment of BKV-infection and prevention/treatment of allograft rejection:

a) Contacting cultivated BKV-infected/ non-infected RPTEC with a portion of whole blood to activate BKV-specific T-cells.
b) Isolating specifically activated T-cells by activation induced-surface markers or activation induced cytokine-secretion. Activated cells are sorted by flow-cytometry or MACS. Specifically activated regulatory T-cells are negatively selected by CD8 T-cell depletion and subsequently positively enriched by CD25 expression (by flow-cytometry or MACS).
c) Isolation success is monitored by flow cytometry.
d) Isolated specific T-cells are cultured *in vitro* for 2-5 weeks with cytokine enriched cell-culture media. Medium for regulatory T-cells supplemented with Interleukin2 (500U/mL) and Rapamycin (100ng/mL). Medium for antigen specific effector supplemented with 50U/mL Interleukin2, 10ng/mL IL7 and/or 10ng/mL IL15.
e) Generated specific TCL are frozen and stored in liquid nitrogen until usage.

The method detailed in Example 1 or 2 can also be used to generate different subtypes of T-cells being specific for antigens derived from BK-virus or graft tissue. For this, the inventors isolated and cultivated epithelial cells as explained above. In order to obtain T-cells specific for BK-virus antigens the cultivated cells were infected with BK-virus and to obtain T-cells specific for graft tissue cultivated epithelial cells were used for the next step.

In details, BKV-specific T-cells can be isolated using activation induced IFNγ-secretion or activation induced surface-markers and expanded for 2-5 weeks in a cell-culture medium containing II-2, IL-7 and/or IL-15 at concentrations of 50U/mL, 10ng/mL and 10ng/mL. The generated BKV-specific T-cell lines can be directly used for the adaptive T-cell therapy in patients with lack of BKV-specific T-cell immunity or frozen and stored in liquid nitrogen for a later usage.

Allograft-reactive regulatory T-cells can be isolated using surface markers CD25, CD127 CD137 and CD154 and expanded for 2-5 weeks in a cell-culture medium containing Interleukin 2 and Rapamycin at concentrations of 500U/mL and 100ng/mL, respectively. The generated allograft-reactive T-cell lines can be directly used for the adaptive T-cell therapy in patients with increased risk of allograft rejection or frozen and stored in liquid nitrogen for a later usage.

### Direct stimulation of T-cells

Direct stimulation was performed for the detection of BKV-specific T-cells as well as alloreactive T-cells. T-cells specific for BK-virus derived antigens presented in a MHC class I context were obtained by incubation of the BK-virus infected epithelial cells in the presence of a whole blood sample from the same patient. The epithelial cells derived from urine as explained above express MHC class I and MHC class II molecules and are therefore suitable for the stimulation in context of MHC class I and II molecules. They are advantageous over other antigen presenting cells derived from the patient, because they are derived from the tissue graft and therefore present antigens in the context of the donor specific MHC molecules. Any T-cells comprised in the blood sample that are specific for BK-virus derived antigens were stimulated by those antigens presented on the cell surface of the BK-virus infected donor- derived epithelial cells. Stimulation of the T-cells with the specific antigen initiated proliferation of these T-cells. Thereby the proliferating T-cells in the following expansion step were mainly T-cells stimulated by their respective antigen.

In order to obtain T-cells specific for the kidney graft tissue the same procedure as detailed above for BK-virus derived antigens was used with the exception of using cultivated epithelial cells as described in the examples above without infection with BK-virus.

### Indirect stimulation of T-cells

Indirect stimulation of T-cells was used to assess alloreactive T-cells. In this alternative approach, T-cells specific for kidney graft tissue derived antigens presented in a MHC class II context of recipient are obtained. Therefore antigen-presenting cells (APCs) expressing MHC class II molecules are isolated from a patient. As explained above, lysed epithelial cells derived from urine of the patient are of donor origin and can be used to be presented in context of recipient APCs. Therefore isolated recipient APCs are incubated with lysed epithelial cells, resulting in the uptake of lysed donor-derived antigens by the APCs. Such antigens are processed by the APCs and are presented in a MHC class II context. Incubating these antigen presenting cells with blood samples comprising T-cells, initiated proliferation of the T-cells recognizing their respective antigen.

T-cells directly or indirectly stimulated are expanded under suitable conditions.

## Claims

1. A method for detecting an immune response in a patient having undergone kidney transplantation, wherein the method comprises the following steps:
a) a cell isolation step comprising:
i. isolating epithelial cells from the urine of said patient,
ii. providing a sample of whole blood obtained from said patient,
iii. dividing said sample of whole blood into a first and a second portion of whole blood,
b) a cell cultivation step comprising:
i. cultivating said epithelial cells, yielding cultivated epithelial cells,
ii. dividing said cultivated epithelial cells into a first and a second portion of cultivated epithelial cells,
iii. contacting said first portion of cultivated epithelial cells with BK-Virus (BKV), yielding BKV-infected epithelial cells,
c) a reactive T-cell determination step, comprising a direct reactive T-cell determination comprising:
i. contacting said sample of whole blood of said patient with said BKV-infected epithelial cells, and
ii. determining T-cells comprised within said sample of whole blood that are activated by said BKV-infected epithelial cells, yielding a BKV-reactive T-cell number;
iii. contacting said second portion of whole blood with said second portion of cultivated epithelial cells and determining T-cells comprised within said second portion of whole blood that are activated by said cultivated epithelial cells, yielding an allograft reactive T-cell number;
wherein the presence of BKV-reactive T-cells is indicative of an anti-BK-virus immune response and the presence of allograft reactive T-cells is indicative of an anti-graft immune response.

2. The method according to claim 1, wherein said epithelial cells isolated from the urine in said cell isolation step:
- only comprises epithelial cells originating from the transplanted kidney of said patient (donor tissue), and/or
- are not infected with BK-virus.

3. The method according to any one of the preceding claims, wherein said T-cells are CD4 or CD8 positive T-cells.

4. The method according to any one of the preceding claims, wherein in step a) PBMCs isolated from blood of the same patient are provided instead of said sample of whole blood.

5. A diagnostic method for monitoring immunosuppressive therapy in a patient after kidney transplantation, comprising determination of said allograft-reactive T-cells and said BK-virus reactive T-cells according to the method of any one of claims 1 to 4, wherein:
- the presence of allograft-reactive T-cells is indicative of a necessary increase in immunosuppressive therapy in said patient, and
- a lack of BKV-reactive T-cells and the detection of a BK-virus infection in said patient is indicative of a necessary reduction in immunosuppressive therapy in said patient.

6. The diagnostic method according to claim 5, wherein:
- allograft reactive T-cells are considered to be present if the allograft-reactive T-cell number is higher than 0.001% of all analysed T-cells,
- BKV-reactive T-cells are considered to be present if the BKV-reactive T-cell number is higher than 0.001 % of all analysed T-cells.

7. The diagnostic method according to any one of claims 5 or 6, wherein said allograft-reactive T-cell number and said BKV-reactive T-cell number are determined at a first and a second time point, wherein
- an increase in said allograft-reactive T-cell number between said first time point and said second time point is indicative of a necessary increase in immunosuppressive therapy, and
- a lack or decrease in BKV-reactive T-cell number between said first and said second timepoint in a patient with a BK-virus infection is indicative of a necessary reduction in immunosuppressive therapy.

8. An *in vitro* method for obtaining a T-cell from a patient after kidney transplantation, wherein said T-cell is specific for antigens derived from:
- BK-virus, or
- kidney graft tissue,
said method comprising the following steps:
a) a cell isolation step comprising:
i. isolating epithelial cells from the urine of said patient,
ii. providing a sample of whole blood obtained from said patient,
b) a cell cultivation step comprising:
i. cultivating said epithelial cells, yielding cultivated epithelial cells,
ii. in case of the antigen being derived from BK-virus, contacting said cultivated epithelial cells with BK-Virus, yielding BKV-infected epithelial cells,
c) a reactive T-cell stimulation step, comprising a direct reactive T-cell stimulation comprising the steps of:
i. contacting said sample of whole blood of said patient with:
- said BKV-infected epithelial cells, in case of the antigen being derived from BK-virus, or
- said cultivated epithelial cells, in case of the antigen being derived from kidney graft tissue,
wherein said T-cell comprised within said sample of whole blood is stimulated with said respective antigen, yielding a stimulated T-cell,
ii. expanding said stimulated T-cell.

9. The method according to claim 8, wherein the obtained T-cell comprises the surface markers selected from:
i. CD4+CD25+Foxp3+CD137+CD154-,
ii. CD8+CD25+Foxp3+,
iii. CD4+RORgt+,
iv. CD4+IL22+IL17-,
v. CD4+CD137+CD154+,
vi. CD4+CD137+GranzymeB+, and
vii. CD8+CD137+GranzymeB+.

## Patentansprüche

1. Ein Verfahren zum Nachweisen einer Immunantwort in einem Patienten, der einer Nierentransplantation unterzogen wurde, wobei das Verfahren die folgenden Schritte umfasst:
a) einen Zellisolierungsschritt, umfassend:
i. Isolieren von Epithelzellen aus dem Urin von besagtem Patienten,
ii. Bereitstellen einer von besagtem Patienten gewonnenen Vollblutprobe,
iii. Aufteilen besagter Vollblutprobe in einen ersten und einen zweiten Teil von Vollblut,
b) einen Zellkultivierungsschritt, umfassend:
i. Kultivieren besagter Epithelzellen, wodurch kultivierte Epithelzellen gewonnen werden,
ii. Aufteilen besagter kultivierter Epithelzellen in einen ersten und einen zweiten Teil von kultivierten Epithelzellen,
iii. Inkontaktbringen besagten ersten Teils von kultivierten Epithelzellen mit BK-Virus (BKV), wodurch BKV-infizierte Epithelzellen gewonnen werden,
c) ein reaktiver T-Zell-Bestimmungsschritt, umfassend eine direkte reaktive T-Zell-Bestimmung, umfassend:
i. Inkontaktbringen besagter Vollblutprobe von besagtem Patienten mit besagten BKV-infizierten Epithelzellen, und
ii. Bestimmen der T-Zellen, umfasst in besagter Vollblutprobe, die durch besagte BKV-infizierte Epithelzellen aktiviert werden, wodurch eine BKV-reaktive T-Zell-Anzahl gewonnen wird;
iii. Inkontaktbringen besagten zweiten Teils der Vollblutprobe mit besagtem zweiten Teil von kultivierten Epithelzellen und Bestimmen der T-Zellen, die in besagtem zweiten Teil der Vollblutprobe umfasst sind, die durch besagte kultivierte Epithelzellen aktiviert werden, wodurch eine Allotransplantat-reaktive T-Zell-Anzahl gewonnen wird;
wobei das Vorhandensein von BKV-reaktiven T-Zellen auf eine Anti-BK-Virus-Immunreaktion und das Vorhandensein von Allotransplantat-reaktiven T-Zellen auf eine Anti-Transplantat-Immunreaktion hinweist.

2. Das Verfahren gemäß Anspruch 1, wobei besagte Epithelzellen, isoliert aus dem Urin in besagtem Zellisolierungsschritt:
- nur Epithelzellen umfasst, die von der transplantierten Niere von besagtem Patienten (Spendergewebe) stammen, und/oder
- nicht mit dem BK-Virus infiziert sind.

3. Das Verfahren gemäß einem der vorhergehenden Ansprüche, wobei besagte T-Zellen CD4 oder CD8 positive T-Zellen sind.

4. Das Verfahren gemäß einem der vorhergehenden Ansprüche, wobei in Schritt a) PBMCs, die aus dem Blut desselben Patienten isoliert wurden, anstelle von besagter Vollblutprobe bereitgestellt werden.

5. Ein diagnostisches Verfahren zum Überwachen einer immunsuppressiven Therapie bei einem Patienten nach einer Nierentransplantation, umfassend die Bestimmung von besagten Allotransplantat-reaktiven T-Zellen und besagten BK-Virus-reaktiven T-Zellen gemäß dem Verfahren nach einem der Ansprüche 1 bis 4, wobei:
- das Vorhandensein von Allotransplantat-reaktiven T-Zellen auf eine notwendige Erhöhung der immunsuppressiven Therapie bei besagtem Patienten hinweist, und
- ein Fehlen von BKV-reaktiven T-Zellen und der Nachweis einer BK-Virusinfektion bei besagtem Patienten ein Hinweis für eine notwendige Reduzierung der immunsuppressiven Therapie bei besagtem Patienten ist.

6. Das diagnostische Verfahren gemäß Anspruch 5, wobei:
- Allotransplantat-reaktive T-Zellen als vorhanden gelten, wenn die Allotransplantat-reaktive T-Zell-Anzahl mehr als 0,001 % aller analysierten T-Zellen beträgt,
- BKV-reaktive T-Zellen als vorhanden gelten, wenn die BKV-reaktive T-Zell-Anzahl mehr als 0,001 % aller analysierten T-Zellen beträgt.

7. Das diagnostische Verfahren gemäß einem der Ansprüche 5 oder 6, wobei die Allotransplantat-reaktive T-Zell-Anzahl und die BKV-reaktive T-Zell-Anzahl zu einem ersten und einem zweiten Zeitpunkt bestimmt werden, wobei
- ein Anstieg der Allotransplantat-reaktiven T-Zell-Anzahl zwischen besagtem ersten Zeitpunkt und besagtem zweiten Zeitpunkt auf eine notwendige Erhöhung der immunsuppressiven Therapie hinweist, und
- ein Fehlen oder eine Abnahme der BKV-reaktiven T-Zell-Anzahl zwischen besagtem ersten und besagtem zweiten Zeitpunkt bei einem Patienten mit einer BK-Virus-Infektion auf eine notwendige Reduzierung der immunsuppressiven Therapie hinweist.

8. Ein *in vitro* Verfahren zum Gewinnen einer T-Zelle aus einem Patienten nach einer Nierentransplantation, wobei die T-Zelle spezifisch für Antigene ist, die abgeleitet sind von:
- BK-Virus, oder
- Nierentransplantatgewebe,
wobei besagtes Verfahren die folgenden Schritte umfasst:
a. einen Zellisolierungsschritt, umfassend:
i. Isolieren von Epithelzellen aus dem Urin von besagtem Patienten,
ii. Bereitstellen einer von besagtem Patienten gewonnenen Vollblutprobe,
b. einen Zellkultivierungsschritt, umfassend:
i. Kultivieren besagter Epithelzellen, wodurch kultivierte Epithelzellen gewonnen werden,
ii. im Falle, dass das Antigen vom BK-Virus abgeleitet ist, Inkontaktbringen besagter kultivierter Epithelzellen mit BK-Virus, wodurch BKV-infizierte Epithelzellen gewonnen werden,
c. einen reaktiven T-Zell-Stimulationsschritt, umfassend eine direkte reaktive T-Zell-Stimulation, umfassend die Schritte:
i. Inkontaktbringen besagter Vollblutprobe von besagtem Patienten mit
- besagten BKV-infizierten Epithelzellen, im Falle, dass das Antigen vom BK-Virus abgeleitet ist, oder
- besagten kultivierten Epithelzellen, im Falle, dass das Antigen aus Nierentransplantatgewebe stammt,
wobei besagte T-Zelle, die in besagter Vollblutprobe umfasst ist, mit besagtem jeweiligen Antigen stimuliert wird, wodurch eine stimulierte T-Zelle gewonnen wird,
ii. Ausdehnen besagter stimulierter T-Zelle.

9. Das Verfahren gemäß Anspruch 8, wobei die gewonnene T-Zelle die Oberflächenmarker umfasst, ausgewählt aus:
a) CD4+CD25+Foxp3+CD137+CD154-,
b) CD8+CD25+Foxp3+,
c) CD4+RORgt+,
d) CD4+IL22+IL 17-,
e) CD4+CD137+CD154+,
f) CD4+CD137+GranzymeB+, und
g) CD8+CD137+GranzymeB+.

## Revendications

1. Procédé de détection d'une réponse immunitaire chez un patient ayant subi une transplantation rénale, dans lequel le procédé comprend les étapes suivantes :
a) une étape d'isolement cellulaire comprenant :
i. isoler des cellules épithéliales de l'urine dudit patient,
ii. fournir un échantillon de sang entier obtenu auprès dudit patient,
iii. diviser ledit échantillon de sang entier en une première et une seconde portion de sang entier,
b) une étape de mise en culture cellulaire comprenant :
i. mettre en culture lesdites cellules épithéliales, donnant des cellules épithéliales cultivées,
ii. diviser lesdites cellules épithéliales cultivées en une première et une seconde portion de cellules épithéliales cultivées,
iii. mettre en contact ladite première portion de cellules épithéliales cultivées avec le virus BK (BKV), donnant des cellules épithéliales infectées par le BKV,
c) une étape de détermination de lymphocytes T réactifs, comprenant une détermination de lymphocytes T réactifs directe comprenant :
i. mettre en contact ledit échantillon de sang entier dudit patient avec lesdites cellules épithéliales infectées par le BKV, et
ii. déterminer des lymphocytes T compris au sein dudit échantillon de sang entier qui sont activés par lesdites cellules épithéliales infectées par le BKV, donnant un nombre de lymphocytes T réactifs au BKV ;
iii. mettre en contact ladite seconde portion de sang entier avec ladite seconde portion de cellules épithéliales cultivées et déterminer des lymphocytes T compris au sein de ladite seconde portion de sang entier qui sont activés par lesdites cellules épithéliales cultivées, donnant un nombre de lymphocytes T réactifs à une allogreffe ;
dans lequel la présence de lymphocytes T réactifs au BKV est indicatrice d'une réponse immunitaire anti-virus BK et la présence de lymphocytes T réactifs à une allogreffe est indicatrice d'une réponse immunitaire anti-greffe.

2. Procédé selon la revendication 1, dans lequel lesdites cellules épithéliales isolées de l'urine dans ladite étape d'isolement cellulaire :
- comprennent uniquement des cellules épithéliales provenant du rein transplanté dudit patient (tissu donneur), et/ou
- ne sont pas infectées par le virus BK.

3. Procédé selon l'une quelconque des revendications précédentes, dans lequel lesdits lymphocytes T sont des lymphocytes CD4 ou CD8 positifs.

4. Procédé selon l'une quelconque des revendications précédentes, dans lequel à l'étape a), des PBMC isolées du sang du même patient sont fournies au lieu dudit échantillon de sang entier.

5. Procédé diagnostique de surveillance de thérapie immunosuppressive chez un patient après transplantation rénale, comprenant la détermination desdits lymphocytes T réactifs à une allogreffe et desdits lymphocytes T réactifs au virus BK conformément au procédé selon l'une quelconque des revendications 1 à 4, dans lequel :
- la présence de lymphocytes T réactifs à une allogreffe est indicatrice d'une augmentation nécessaire de la thérapie immunosuppressive chez ledit patient, et
- un manque de lymphocytes T réactifs au BKV et la détection d'une infection au virus BK chez ledit patient sont indicateurs d'une réduction nécessaire de la thérapie immunosuppressive chez ledit patient.

6. Procédé diagnostique selon la revendication 5, dans lequel :
- des lymphocytes T réactifs à une allogreffe sont considérés comme étant présents si le nombre de lymphocytes T réactifs à une allogreffe est supérieur à 0,001 % de tous les lymphocytes T analysés,
- des lymphocytes T réactifs au BKV sont considérés comme étant présents si le nombre de lymphocytes T réactifs au BKV est supérieur à 0,001 % de tous les lymphocytes T analysés.

7. Procédé diagnostique selon l'une quelconque des revendications 5 ou 6, dans lequel ledit nombre de lymphocytes T réactifs à une allogreffe et ledit nombre de lymphocytes T réactifs au BKV sont déterminés à un premier et un second instant, dans lequel
- une augmentation dudit nombre de lymphocytes T réactifs à une allogreffe entre ledit premier instant et ledit second instant est indicatrice d'une augmentation nécessaire de la thérapie immunosuppressive, et
- un manque ou une diminution du nombre de lymphocytes T réactifs au BKV entre ledit premier et ledit second instant chez un patient avec une infection au virus BK est indicateur d'une réduction nécessaire de la thérapie immunosuppressive.

8. Procédé *in vitro* d'obtention d'un lymphocyte T chez un patient après transplantation rénale, dans lequel ledit lymphocyte T est spécifique pour des antigènes dérivés de :
- virus BK, ou
- tissu de greffe rénal,
ledit procédé comprenant les étapes suivantes :
a) une étape d'isolement cellulaire comprenant :
i. isoler des cellules épithéliales de l'urine dudit patient,
ii. fournir un échantillon de sang entier obtenu auprès dudit patient,
b) une étape de mise en culture cellulaire comprenant :
i. mettre en culture lesdites cellules épithéliales, donnant des cellules épithéliales cultivées,
ii. au cas où l'antigène est dérivé du virus BK, mettre en contact lesdites cellules épithéliales cultivées avec le virus BK, donnant des cellules épithéliales infectées par le BKV,
c) une étape de stimulation de lymphocytes T réactifs, comprenant une stimulation de lymphocytes T réactifs directe comprenant les étapes de :
i. mise en contact dudit échantillon de sang entier dudit patient avec :
- lesdites cellules épithéliales infectées par le BKV, au cas où l'antigène est dérivé du virus BK, ou
- lesdites cellules épithéliales cultivées, au cas où l'antigène est dérivé de tissu de greffe rénal,
dans lequel ledit lymphocyte T compris au sein dudit échantillon de sang entier es stimulé avec ledit antigène respectif, donnant un lymphocyte T stimulé,
ii. réaliser une expansion dudit lymphocyte T stimulé.

9. Procédé selon la revendication 8, dans lequel le lymphocyte T obtenu comprend les marqueurs de surface sélectionnés parmi :
i. CD4+CD25+Foxp3+CD137+CD154-,
ii. CD8+CD25+Foxp3+,
iii. CD4+RORgt+,
iv. CD4+IL22+IL17-,
v. CD4+CD137+CD154+,
vi. CD4+CD137+GranzymeB+, et
vii. CD8+CD137+GranzymeB+.
